# EUROPEAN PATENT SPECIFICATION

(11) **EP 2 254 528 B1**
(45) Date of publication and mention of the grant of the patent: **26.10.2016**
(21) Application number: 08872582.5
(22) Date of filing: 21.11.2008
(51) Int. Cl.: A61F 2/95

(54) **HANDLE ASSEMBLY FOR A DELIVERY SYSTEM**
HANDGRIFFANORDNUNG FÜR EIN ABGABESYSTEM
POIGNÉE POUR SYSTÈME D'ADMINISTRATION

(30) Priority: 20.02.2008 US 34080
(43) Date of publication of application: 01.12.2010
(73) Proprietor: ABBOTT LABORATORIES, Abbott Park, IL 60064-3500 (US)
(72) Inventor: SHUMER, Daniel H., San Jose, California 95123 (US); OSMAN, Karim S., Mountain View, California 94040 (US); YOUNG, Eugene, Union City, California 94587 (US); ORTEGA, Leopoldo, Murrieta, California 92562 (US)
(74) Representative: Boult Wade Tennant
(86) International application number: PCT/US2008/084356
(87) International publication number: WO 2009/105136

(56) References cited:
- WO-A-2006/133959
- WO-A-2007/022395
- WO-A-2009/012061
- DE-A1-102006 004 123
- US-A1- 2007 168 014

## Description

### BACKGROUND OF THE INVENTION

The present invention relates generally to handles for medical device delivery systems and, more particularly, to a handle for a vascular stent delivery system.

Stents are generally cylindrically shaped devices which function to hold open and sometimes expand a segment of a blood vessel or other arterial lumen, such as coronary artery. Stents are usually delivered in a compressed condition to the target site and then deployed at that location into an expanded condition to support the vessel and help maintain it in an open position. They are particularly suitable for use to support and hold back a dissected arterial lining which can occlude the fluid passageway there through. Stents are particularly useful in the treatment and repair of blood vessels after a stenosis has been compressed by percutaneous transluminal coronary angioplasty, percutaneous transluminal angioplasty, or removed by atherectomy or other means, to help improve the results of the procedure and reduce the possibility of restenosis. Stents, or stent like devices, are often used as the support and mounting structure for implantable vascular grafts which can be used to create an artificial conduit to bypass the diseased portion of the vasculature, such as an abdominal aortic aneurism.

A variety of devices are known in the art for use as stents and have included coiled wires in a variety of patterns that are expanded after being placed intraluminally on a balloon catheter, helically wound coiled springs manufactured from an expandable heat sensitive metal; and self expanding stents inserted into a compressed state for deployment into a body lumen. One of the difficulties encountered in using prior art stents involve maintaining the radial rigidity needed to hold open a body lumen while at the same time maintaining the longitudinal flexibility of the stent to facilitate its delivery and accommodate the often tortuous path of the body lumen.

Prior art stents typically fall into two general categories of construction. The first type of stent is expandable upon application of a controlled force, often through the inflation of the balloon portion of a dilatation catheter which, upon inflation of the balloon or other expansion means, expands the compressed stent to a larger diameter to be left in place within the artery at the target site. The second type of stent is a self expanding stent formed from shape memory metals or superelastic nickel titanium alloys, which will automatically expand from a compressed state when the stent is advanced out of the distal end of the delivery device, or when a restraining sheath which holds the compressed stent in its delivery position is retracted to expose the stent.

Some prior art stent delivery systems for delivery and implanting self expanding stents include an inner member upon which the compressed or collapsed stent is mounted and an outer restraining sheath which is initially placed over the compressed stent prior to deployment. When the stent is to be deployed in the body vessel, the outer sheath is moved in relation to the inner member to "uncover" the compressed stent, allowing the stent to move to its expanded condition. Some delivery systems utilize a "push pull" type technique in which the outer sheath is retracted while the inner member is pushed forward. Another common delivery system utilizes a simple pull back delivery system in which the self expanding stent is maintained in its compressed position by an outer sheath. Once the mounted stent has been delivered to the desired treatment location, the outer sheath is pulled back via a deployment handle located at a remote position outside of the patient, which uncovers the stent to allow it to self expand within the patient. Still other delivery systems use an actuating wire attached to the outer sheath. When the actuating wire is pulled to retract the outer sheath and deploy the stent, the inner member must remain stationary, preventing the stent from moving axially within the body vessel.

Controlled deployment of the stent can be a desirable feature in various applications. This can be particularly true when attempting to deploy a self-expanding stent which may tend to spring forwardly when withdrawing the sheath. Further, it can be desirable to employ a system which provides such control.

For example, in US 2007/168014 there is described a stent delivery system which includes a retractable sheath and an outer stability sheath. The stability sheath freely rotates relative to the retractable sheath, relieving compression forces caused by twisting of the stability sheath when in a tortuous conformation.

By contrast, in DE 10 2006 004123 there is described a device for inserting a self-expanding stent into a body vessel. The device comprises a tube, a pushing element and a grip having a housing via which the pushing element is secured on the grip. Further, the device comprises a stent carrier and a movable element, the latter of which is guided in the housing of the grip and is coupled to the pushing element. Moving the movable element in the proximal direction effects a movement of the pushing element in the distal direction and a movement of the tube in the proximal direction.

It has been found to be desirable to have a handle for a delivery system which provides additional control. It has been contemplated that a handle including multiple structures providing varied control may address this need.

The present invention disclosed herein satisfies these and other needs.

### SUMMARY OF THE INVENTION

Briefly and in general terms, the present invention is directed towards a system for delivering a medical device within vasculature.

According to the present invention the system comprises the features set out in claim 1.

In one aspect, the medical device is a self-expanding stent.

In one embodiment, the handle assembly includes a plurality of sub-assemblies which cooperate to accomplish the delivery of a medical device within a patient's body. The handle assembly can include a trigger assembly and a thumbwheel assembly each of which are operatively associated with a shuttle assembly. A locking mechanism can be further provided to lock these assemblies in place prior to use.

In a particular aspect, the present invention includes a handle assembly including a belt attached at one end to a shuttle assembly and configured about a thumbwheel spool at its other end. The shuttle assembly is configured to move longitudinally with respect to a handle casing of the handle assembly. The shuttle assembly is further attached to a sheath assembly enclosing the medical device. Further, the shuttle assembly can be activated in a plurality of ways. That is, the shuttle assembly can be manipulated via a trigger assembly operatively connected to the shuttle and can alternatively be translated by rotating the thumbwheel assembly which includes the thumbwheel spool.

In a further aspect of the present invention, the belt has a thickness and a width, the width being greater than the thickness. The belt is configured generally vertically at its connection to the shuttle assembly which is slideably configured within a distal portion of the handle assembly. From this connection, the belt extends proximally past a first side of the thumbwheel assembly and around a proximal spool. The belt is directed distally and is turned counterclockwise to assume a horizontal configuration when viewing the belt from a proximal location. While horizontal, the belt passes a second side of the thumbwheel assembly, extending distally to and about a pair of idler trigger sleeves and a pulley trigger then back proximally to the spool of the thumbwheel assembly.

Accordingly, the present invention provides dual methods of the withdrawal of a sheath of the delivery system. In this manner, the operator is provided with enhanced control of the delivery and implantation of a medical device.

These and other features of the present invention become apparent from the following detailed description and the accompanying exemplary drawings.

### BRIEF DESCRIPTION OF THE DRAWINGS

FIG. 1 is a perspective view, depicting a right side view of a delivery system of the present invention;
FIG. 2 is a perspective view, depicting the delivery system of FIG. 1 with a right handle casing removed;
FIG. 3 is a perspective view, depicting a left side view of a delivery system of the present invention;
FIG. 4 is a perspective view, depicting the delivery system of FIG. 3 with a left handle casing removed;
FIG. 5 is a perspective view, depicting belt and shuttle assemblies of the delivery system of FIG. 1;
FIG. 6 is a perspective view, depicting thumbwheel and guide rail assemblies of the delivery system of FIG. 1;
FIG. 7 is an enlarged view, depicting a belt and shuttle connection with various other components removed for ease of illustration;

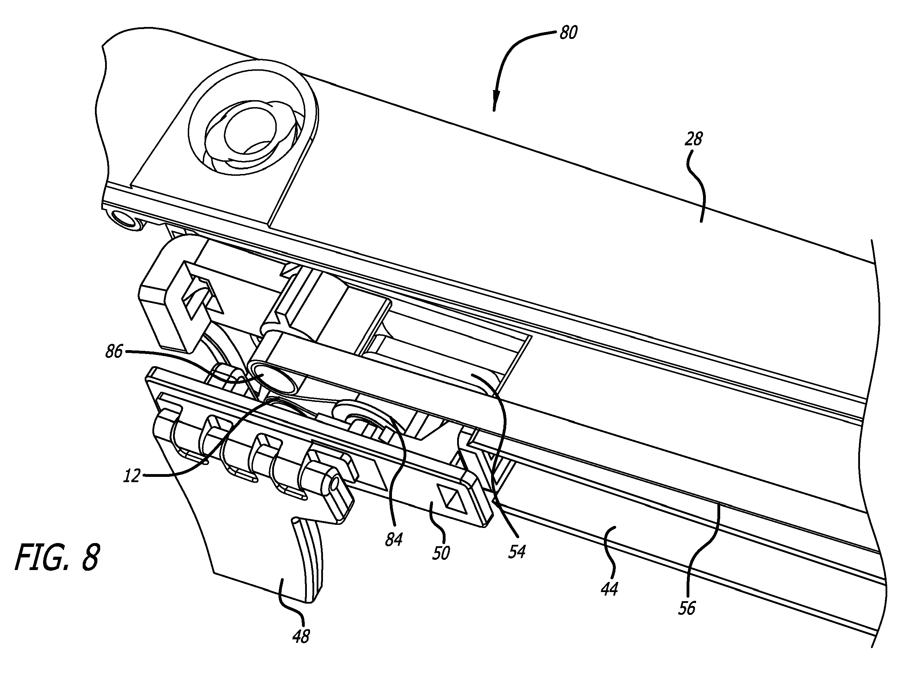

FIG. 8 is an enlarged view, depicting components cooperating with the trigger assembly with various other components removed for ease of illustration; and
FIG. 9 is an enlarged view, depicting a locking mechanism of the delivery system with various other components removed for ease of illustration.

### DETAILED DESCRIPTION OF THE PREFERRED EMBODIMENTS

The present invention relates to a system that delivers and deploys a medical device at a target site within a patient's body, such as a body lumen. For illustration purposes, the following exemplary embodiments are directed to a handle assembly for a system for delivering and deploying a self expanding stent, although it is understood that the present invention is applicable to other medical devices which are implantable in a body lumen as well as other parts of the body. Additionally, the medical device can be either a selfexpanding device or a non self-expanding device.

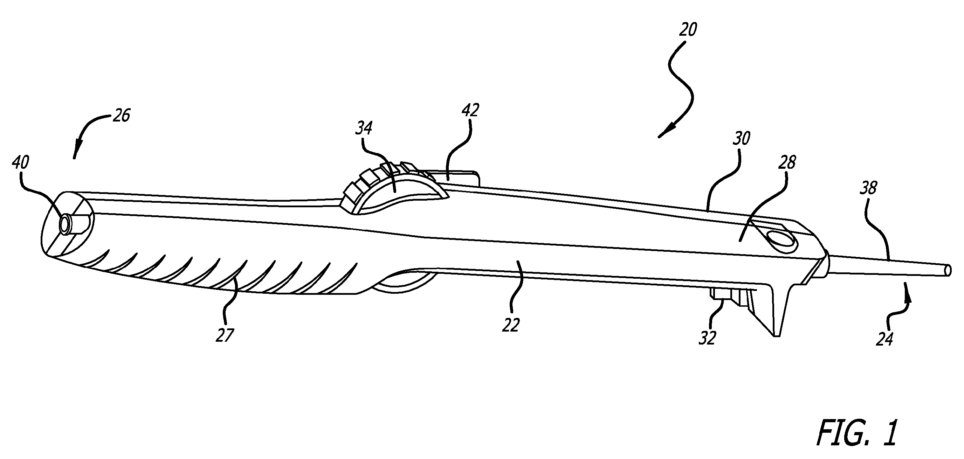

Referring now to FIGS. 1-4, a system 20 incorporating a handle assembly 22 of the present invention is illustrated. The handle assembly 22 includes a superior or distal end portion 24 and an inferior or proximal end portion 26. The handle assembly 22 is generally elongate and includes a gripping portion 27 that comfortably fits in an operator's hand. Additionally, encasing internal components of the handle assembly are a first handle housing 28 which mates with a second handle housing 30.

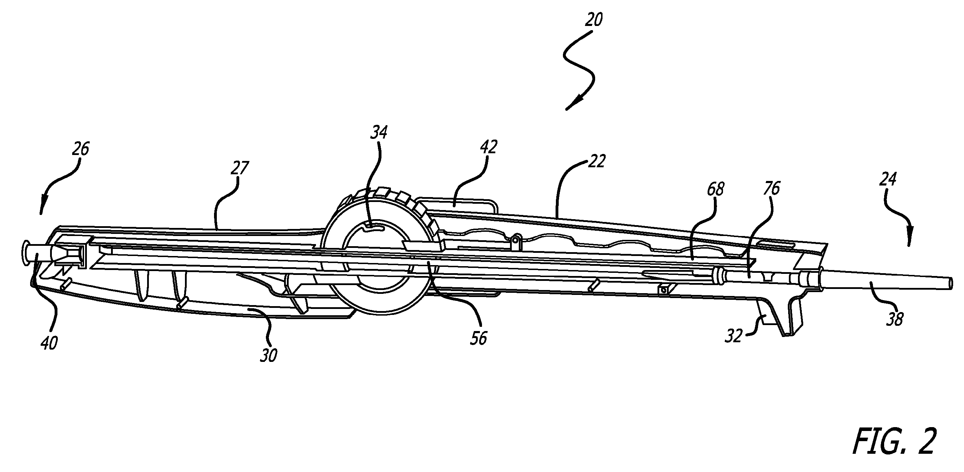

Further, in one aspect, the handle assembly includes a trigger sub-assembly 32 and a thumbwheel sub-assembly 34 each of which are mounted to or within the handle housings 28, 30 and which can be alternatively actuated to effect longitudinal movement of a shuttle assembly 36 (See FIG. 2). The thumbwheel assembly 34 is conveniently located at a midsection of the handle assembly 22 so that an operator can use the thumb of the hand holding the gripping portion 27 to actuate the thumbwheel assembly 34. Actuation of the trigger sub-assembly 32 can be manipulated by the operator's other hand. The distal end portion 24 of the handle assembly 22 is further configured with a strain relief device 38. The proximal end portion 26 further includes a luer fitting 40. A channel or space is provided between the luer fitting 40 and strain relief device 38 for receiving other portions of a delivery system 20 such as a lumen (not shown) for receiving longitudinal members such as a guide wire.

The system 20 can further be equipped with a locking mechanism 42. This locking mechanism 42 cooperates both with the thumbwheel sub-assembly 34 and the shuttle subassembly 36 to inhibit activation of those assemblies when the device is not in use.

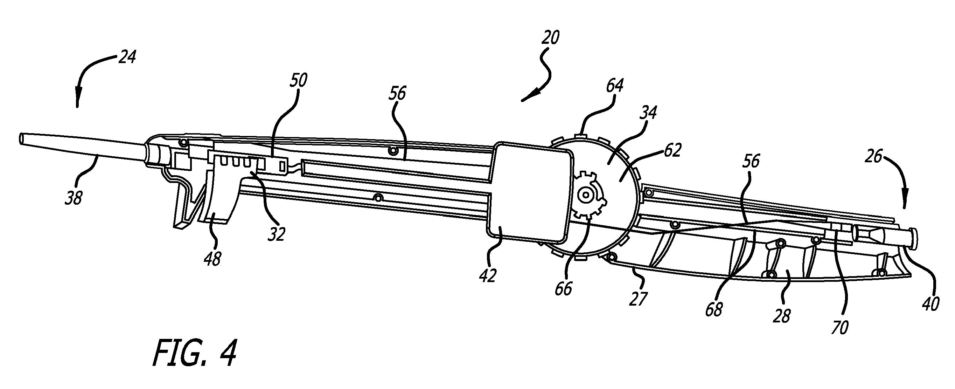

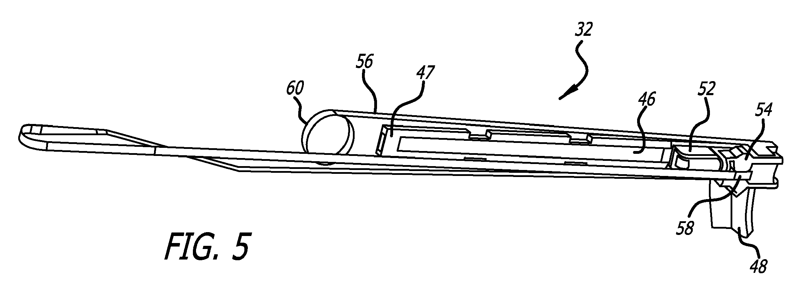

Turning now to FIG. 5, along with reference to FIG. 4, components of the trigger sub-assembly 32 are identified. Various of the components of the trigger sub-assembly 32 are supported by a trigger retainer assembly 44. The trigger retainer assembly 44 is generally rectangular in shape and includes a central cavity 46 forming a channel along which components of the trigger sub-assembly 32 can be translated longitudinally there along and within the handle assembly 22. The trigger retainer assembly 44 further includes various indentations and recesses for securely mounting the assembly within the handle housings 28, 30.

Furthermore, the trigger sub-assembly 32 includes a trigger handle 48 which is connected to a trigger sliding component 50 (See FIG. 4). The trigger slider 50 mates with a pulley retainer 52 and is sized and shaped to move longitudinally along the trigger retainer assembly 44. The pulley retainer 52, in turn, engages an idler retainer 54 when the trigger handle 48 is located in its most forward position (See FIG. 5).

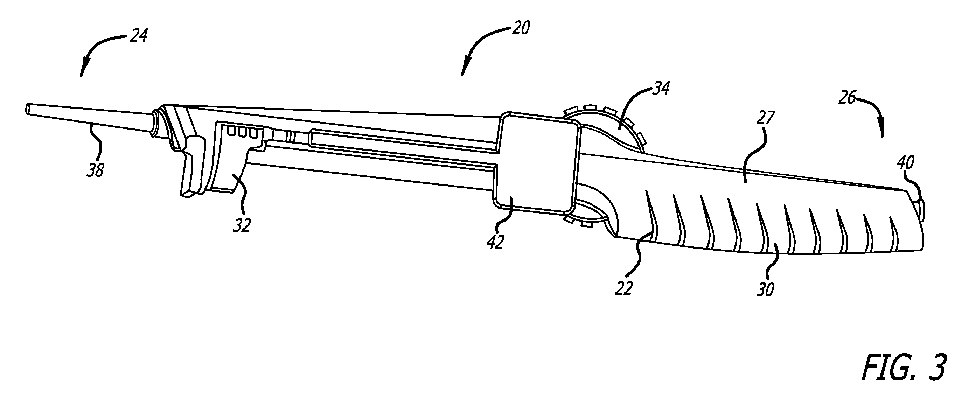

The trigger sub-assembly further including a belt 56 which extends back and forth through a plurality of turns within the handle assembly 22. The belt 56 has a thickness and a width, the width being greater than the thickness. In one aspect, the belt 56 has a generally constant width and has generally orthogonally arranged surfaces. As described in more detail below, one end 58 of the belt 56 is attached to the shuttle assembly 36 (See FIG. 3) and another end 60 is wrapped about a thumbwheel pulley.

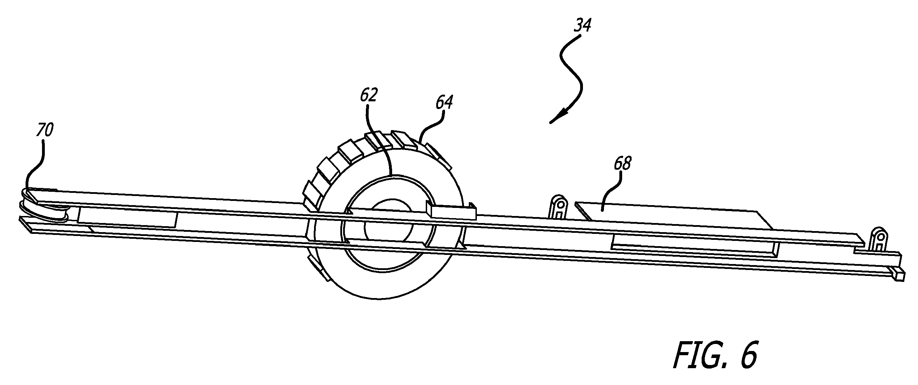

As shown in FIG. 6, with continued reference to FIG. 4, the thumbwheel sub-assembly 34 includes a thumbwheel 62 about a circumference thereof is configured a thumbwheel overmold 64. The overmold 64 can be formed of elastomeric or similar material which makes the assembly easy to manipulate. As best seen in FIG. 4, a center portion of the thumbwheel is equipped with a thumbwheel pulley 66 about which the belt gathers upon actuation or rotation of the thumbwheel 62.

Moreover, the thumbwheel sub-assembly 34 includes an elongate, thumbwheel pivot web assembly 68. The pivot web assembly 68 defines a rail-like sub-structure which extends substantially a length of the handle assembly 22 (See FIG. 4) and includes various indentations and recesses for mounting structure thereto and for affixing the assembly within the handle housings 28, 30. At a proximal end of the pivot web assembly 68 there is configured a proximal spool 70 held between opposing members so that it can rotate in place. A mid section of the web 68 includes structure about which the thumbwheel 62 is supported and can rotate.

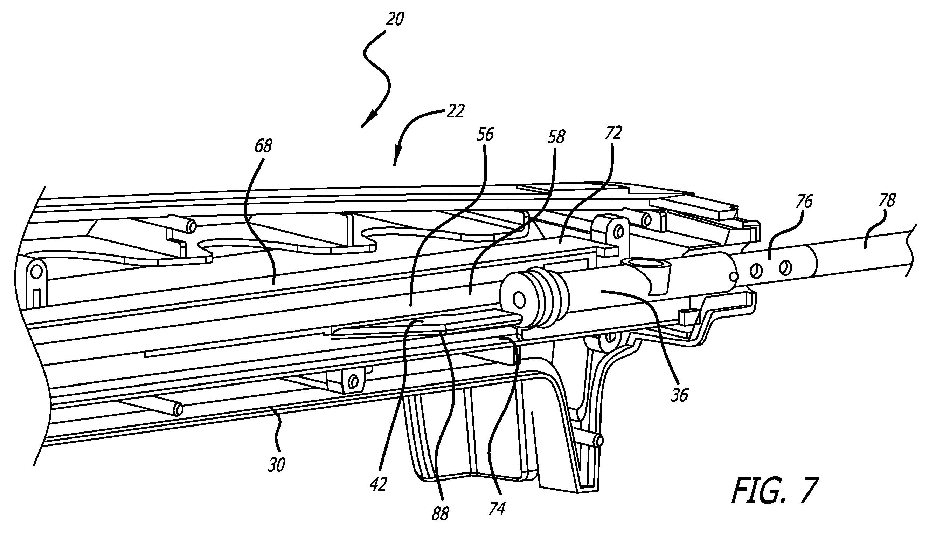

With reference now to FIG. 7, the shuttle assembly 36 is shown in its most distal configuration. The shuttle assembly 36 is slideably received between opposing, spaced members 72, 74 of the pivot web assembly 68. The distal terminal end portion 58 of the belt 56 is attached to an internal surface of the shuttle assembly 36. A distal end portion 76 of the shuttle assembly 36 can be attached to a sheath 78 or other structure to be withdrawn to thereby deliver a medical device (not shown) within a patient's body. At its distal terminal end 58, the belt is configured vertically at its attachment to the shuttle assembly 36.

From its distal terminal end connection to the shuttle assembly 36, the belt 56 extends proximally. As best seen in FIG. 5 along with reference to FIG. 4, the belt 56 continues in its generally vertical configuration proximally to and about the proximal spool 70. The belt 56 then changes direction and proceeds distally for a length. The belt 56 then makes a ninety degree (90°) counterclockwise turn (when viewing the assembly from a proximal standpoint) and continues distally in a horizontal fashion.

Upon reaching a distal portion 80 of the handle housing 28, the belt 56 is routed first about a lower idler 82 then proximally again about a trigger pulley 84. The belt 56 takes yet another turn of direction distally and is routed about an upper idler 86. From here, the belt 56 extends proximally once again in the direction of the thumbwheel sub-assembly 34 (See also FIG. 4). Upon reaching the thumbwheel sub-assembly 34, the belt is received about the thumbwheel spool 66.

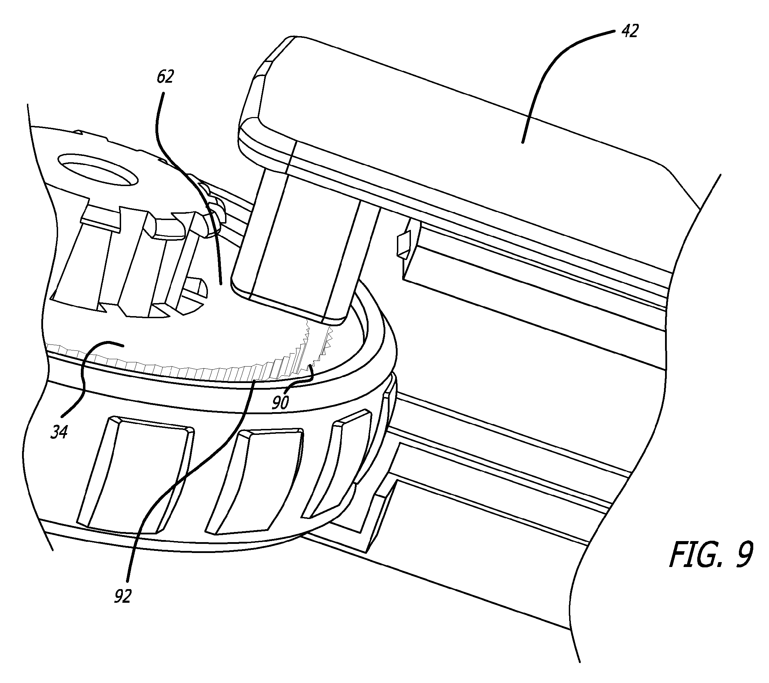

Prior to use, the locking mechanism 42 is placed into engagement with both the shuttle assembly 36 and thumbwheel sub-assembly 34 (See FIGS. 7 and 9). A distally configured wing 88 of the locking mechanism is placed into engagement with a proximal face of the shuttle assembly 36. A transversely extending locking tab 90 configured at a proximal end portion of the locking mechanism 42 is placed within a circular recess 92 formed in one side of the thumbwheel 62. In this way, the locking mechanism 42 restrains the movement of both the shuttle assembly 36 and the thumbwheel sub-assembly 34.

As stated, in use, either the thumbwheel sub-assembly 34 or the trigger sub-assembly 32 can be actuated to effect longitudinal relative movement between a sheath or similar structure and a medical device to accomplish deployment of the medical device at a treatment site. Moreover, each of the thumbwheel 34 and trigger 32 sub-assembly can be actuated alternatively or in exclusion of the other to accomplish such deployment.

Through the rotation of the thumbwheel 62, the proximal terminal end 60 of the belt 56 gathers about the thumbwheel spool 66 (See FIG. 4 and 5). This action consequently reduces the extended length of the belt 56 and thereby causes the shuttle assembly 36 to move proximally along the pivot web assembly 68.

Actuation or rather proximal movement of the trigger 48 of the trigger sub-assembly 32 likewise causes the shuttle assembly 36 to move proximally along the pivot web assembly 68. Here, as the trigger is moved proximally, the trigger slider 50 travels proximally along the trigger retainer assembly 44 (See FIGS. 4 and 5). By way of a fixed connection, the trigger slider 50 brings the pulley proximally thereby reconfiguring the pattern through which the belt 56 must travel from its connection to the shuttle assembly 36. Since this path reconfiguration is essentially an attempt to lengthen an otherwise fixed length belt 56, the shuttle assembly 32 is caused to move proximally.

Therefore, the handle assembly of the present invention provides a system including a plurality of means for accomplishing relative motion.

It is to be understood that even though numerous characteristics and advantages of the present invention have been set forth in specific description, together with details of the structure and function of the invention, the disclosure is illustrative only and changes may be made in detail, such as size, shape and arrangement of the various components of the present invention, without departing from the scope of the present invention. It would be appreciated to those skilled in the art that further modifications or improvement may additionally be made to the delivery system disclosed herein without departing from the scope of the invention. Accordingly, it is not intended that the invention be limited, except as by the appended claims.

## Claims

1. A system for delivering a medical device within vasculature, comprising:
a sheath assembly (78);
a medical device retained in the sheath assembly (78); and
a handle assembly (22) including a shuttle assembly (36) connected to the sheath assembly (78), the handle assembly (22) further including a trigger handle (48) connected to a trigger sliding component (50) adapted to move longitudinally within the handle assembly (22), the trigger sliding component (50) mating with a pulley retainer (52) and the pulley retainer (52) in turn engaging an idler retainer (54) when the trigger handle (48) is located in its most forward position, such that the pulley retainer (52) is moveable with the trigger sliding component (50),
the handle assembly (22) further including a belt (56) attached at one end to the shuttle assembly (36) and at another end to a thumbwheel assembly (34), the belt (56)
(i) being arranged vertically at its connection to the shuttle assembly (36) and extending proximally from the shuttle assembly (36) to a proximal spool (70),
(ii) extending distally from the proximal spool (70) and being horizontal along a portion of its length when extending distally from the proximal spool (70),
(iii) being routed about a pair of idlers (82,86) of the idler retainer (54) and a trigger pulley (84) of the pulley retainer (52), and
(iv) extending proximally from the pair of idlers (82, 86) to be connected to the thumbwheel assembly (34),
wherein actuation of either the trigger handle (48) or the thumbwheel assembly (34) accomplishes longitudinal movement of the shuttle assembly (36) within the handle assembly (22).

2. The system of claim 1, wherein the belt (56) is configured both vertically and horizontally.

3. The system of claim 1, wherein the belt (56) is configured to assume a plurality of turns.

4. The system of claim 1, wherein the belt (56) makes four turns.

5. The system of claim 1, further including a locking mechanism (42) which locks both the thumbwheel assembly (34) and the shuttle assembly (36).

6. The system of claim 1, wherein the belt (56) has a width and a thickness, the width being greater than the thickness.

7. The system of claim 1, wherein the handle assembly (22) includes a pair of opposing, spaced apart members (72,74) between which the shuttle assembly (36) is slidably received.

8. The system of claim 7, wherein a portion of the belt (56) is positioned between the pair of opposing, spaced apart members (72,74).

9. The system of claim 1, further including a trigger retaining assembly (44) which is generally rectangular in shape and includes a central cavity (46) forming a channel along which the trigger sliding component (50) can translate longitudinally within the handle assembly (22).

10. The system of claim 1, wherein each of the tumbwheel assembly (34) and the trigger handle (48) can be actuated alternatively or in exclusion of the other to accomplish longitudinal motion of the shuttle assembly (36) within the handle assembly (22).

## Patentansprüche

1. System zum Einbringen eines Medizinproduktes in ein Gefäßsystem, das aufweist:
eine Hüllenbaugruppe (78);
ein Medizinprodukt, das in der Hüllenbaugruppe (78) aufgenommen ist; und
eine Griffbaugruppe (22), die eine mit der Hüllenbaugruppe (78) verbundene Transportbaugruppe (36) aufweist, wobei die Griffbaugruppe (22) ferner einen Auslösehebel (48) aufweist, der mit einer Auslösegleitkomponente (50) verbunden ist, die für eine Längsbewegung innerhalb der Griffbaugruppe (22) ausgebildet ist, wobei die Auslösegleitkomponente (50) mit einem Riemenrollenhalter (52) verbunden ist und der Riemenrollenhalter (52) wiederum in einen Umlenkrollenhalter (54) eingreift, wenn sich der Auslösehebel (48) in seiner vordersten Position befindet, sodass der Riemenrollenhalter (52) zusammen mit der Auslösegleitkomponente (50) bewegbar ist,
wobei die Griffbaugruppe (22) ferner einen Riemen (56) aufweist, der an einem Ende mit der Transportbaugruppe (36) und an dem anderen Ende mit einer Daumenradbaugruppe (34) verbunden ist, wobei der Riemen (56)
(i) an seiner Verbindung mit der Transportbaugruppe (36) vertikal angeordnet ist und sich proximal von der Transportbaugruppe (36) zu einer proximalen Spule (70) erstreckt,
(ii) sich von der proximalen Spule (70) distal erstreckt und entlang eines Teils seiner Länge horizontal verläuft, wenn er sich von der proximalen Spule (70) distal erstreckt,
(iii) über ein Umlenkrollenpaar (82, 86) des Umlenkrollenhalters (54) und eine Auslöseriemenrolle (84) des Riemenrollenhalters (52) geführt wird, und
(iv) sich proximal von dem Umlenkrollenpaar (82, 86) erstreckt, um mit der Daumenradbaugruppe (34) verbunden zu werden,
wobei durch eine Betätigung von entweder des Auslösehebels (48) oder der Daumenradbaugruppe (34) eine Längsbewegung der Transportbaugruppe (36) innerhalb der Griffbaugruppe (22) bewirkt wird.

2. System nach Anspruch 1, wobei der Riemen (56) sowohl vertikal als auch horizontal konfiguriert ist.

3. System nach Anspruch 1, wobei der Riemen (56) so ausgebildet ist, dass er mehrere Drehungen aufweist.

4. System nach Anspruch 1, wobei der Riemen (56) vier Drehungen aufweist.

5. System nach Anspruch 1, das ferner einen Verriegelungsmechanismus (42) aufweist, der sowohl die Daumenradbaugruppe (34) als auch die Transportbaugruppe (36) verriegelt.

6. System nach Anspruch 1, wobei der Riemen (56) eine Breite und eine Dicke aufweist und die Breite größer als die Dicke ist.

7. System nach Anspruch 1, wobei die Griffbaugruppe (22) ein Paar einander gegenüberliegender, zueinander beabstandeter Elemente (72, 74) aufweist, zwischen denen die Transportbaugruppe (36) gleitend aufgenommen ist.

8. System nach Anspruch 7, wobei ein Teil des Riemens (56) zwischen dem Paar einander gegenüberliegender, zueinander beabstandeter Elemente (72, 74) angeordnet ist.

9. System nach Anspruch 1, das ferner eine Auslöserhaltebaugruppe (44) aufweist, die eine in etwa rechteckige Form aufweist und einen zentralen Hohlraum (46) umfasst, der einen Kanal bildet, wobei die Auslösegleitkomponente (50) entlang des Kanals in Längsrichtung innerhalb der Griffbaugruppe (22) verschoben werden kann.

10. System nach Anspruch 1, wobei sowohl die Daumenradbaugruppe (34) als auch der Auslösehebel (48) wahlweise oder unter Ausschluss des anderen betätigt werden kann, um eine Längsbewegung der Transportbaugruppe (36) innerhalb der Griffbaugruppe (22) zu bewirken.

## Revendications

1. Système permettant de délivrer un dispositif médical dans une vasculature, comprenant :
un ensemble gaine (78) ;
un dispositif médical retenu dans l'ensemble gaine (78) ; et
un ensemble poignée (22) comportant un ensemble navette (36) relié à l'ensemble gaine (78), l'ensemble poignée (22) comportant en outre une poignée de déclenchement (48) reliée à un composant coulissant de déclenchement (50) adapté pour se déplacer longitudinalement à l'intérieur l'ensemble poignée (22), le composant coulissant de déclenchement (50) étant accouplé à un élément de retenue de poulie (52) et l'élément de retenue de poulie (52) s'engageant, à son tour, avec un élément de retenue intermédiaire (54) lorsque la poignée de déclenchement (48) se trouve dans sa position la plus avancée, de sorte que l'élément de retenue de poulie (52) puisse se déplacer avec le composant coulissant de déclenchement (50),
l'ensemble poignée (22) comportant en outre une courroie (56) fixée au niveau d'une extrémité à l'ensemble navette (36) et au niveau de l'autre extrémité à un ensemble à molette (34), la courroie (56)
(i) étant agencée verticalement au niveau de sa liaison à l'ensemble navette (36) et s'étendant de manière proximale depuis l'ensemble navette (36) vers une bobine proximale (70),
(ii) s'étendant de manière distale depuis la bobine proximale (70) et étant horizontale le long d'une partie de sa longueur lorsqu'elle s'étend de manière distale depuis la bobine proximale (70),
(iii) étant dirigée entre une paire de rouleaux (82, 86) de l'élément de retenue intermédiaire (54) et une poulie de déclenchement (84) de l'élément de retenue de poulie (52), et
(iv) s'étendant de manière proximale depuis la paire de rouleaux (82, 86) devant être reliée à l'ensemble à molette (34),
où l'actionnement de l'un(e) ou l'autre parmi la poignée de déclenchement (48) et l'ensemble à molette (34) accomplit un mouvement longitudinal de l'ensemble navette (36) dans l'ensemble poignée (22).

2. Système de la revendication 1, dans lequel la courroie (56) est configurée à la fois verticalement et horizontalement.

3. Système de la revendication 1, dans lequel la courroie (56) est configurée pour réaliser une pluralité de tours.

4. Système de la revendication 1, dans lequel la courroie (56) effectue quatre tours.

5. Système de la revendication 1, comportant en outre un mécanisme de verrouillage (42) qui verrouille à la fois l'ensemble à molette (34) et l'ensemble navette (36).

6. Système de la revendication 1, dans lequel la courroie (56) a une largeur et une épaisseur, la largeur étant supérieure à l'épaisseur.

7. Système de la revendication 1, dans lequel l'ensemble poignée (22) comporte une paire d'éléments opposés et espacés (72, 74) entre lesquels l'ensemble navette (36) est reçu en coulissement.

8. Système de la revendication 7, dans lequel une partie de la courroie (56) est positionnée entre la paire d'éléments opposés et espacés (72, 74).

9. Système de la revendication 1, comportant en outre un ensemble élément de retenue de déclenchement (44) qui est de forme globalement rectangulaire et comporte une cavité centrale (46) formant un canal le long duquel le composant coulissant de déclenchement (50) peut faire une translation longitudinalement dans l'ensemble poignée (22).

10. Système de la revendication 1, dans lequel chacun(e) parmi l'ensemble à molette (34) et la poignée de déclenchement (48) peut être actionné(e) alternativement ou à l'exclusion de l'autre pour réaliser un mouvement longitudinal de l'ensemble navette (36) à l'intérieur de l'ensemble poignée (22).
